# EUROPEAN PATENT SPECIFICATION

(11) **EP 1 517 990 B1**
(45) Date of publication and mention of the grant of the patent: **19.03.2008**
(21) Application number: 03761887.3
(22) Date of filing: 26.06.2003
(51) Int. Cl.: C12N 15/10, C07H 1/06, C07H 1/08

(54) **ISOLATION OF NUCLEIC ACIDS USING A POLYCATIONIC POLYMER AS PRECIPITATION AGENT**
ISOLIERUNG VON NUKLEINSÄUREN UNTER VERWENDUNG EINES POLYKATIONISCHEN POLYMERS ALS FÄLLUNGSMITTEL
ISOLATION D'ACIDES NUCLEIQUES A L'AIDE D'UN POLYMERE POLYCATIONIQUE UTILISE EN TANT QU'AGENT DE PRECIPITATION

(30) Priority: 28.06.2002 SE 0202074; 08.04.2003 SE 0301034
(43) Date of publication of application: 30.03.2005
(73) Proprietor: GE Healthcare Bio-Sciences AB, 751 84 Uppsala (SE)
(72) Inventor: GALAEV, Igor Yu, S-221 00 Lund (SE); GUSTAVSSON, Per-Erik, S-221 00 Lund (SE); IZUMRUDOV, Vladimir A, Polymer Chemistry Dpt., 119899 Moscow (RU); LARSSON, Per-Olof, S-221 00 Lund (SE); WAHLUND, Per-Olof, S-221 00 Lund (SE)
(74) Representative: Franks, Barry Gerard
(86) International application number: PCT/SE2003/001127
(87) International publication number: WO 2004/003200

(56) References cited:
- EP-A1- 1 031 626
- EP-A2- 0 281 390
- US-A- 4 055 469
- US-A- 5 010 183
- US-A1- 2002 010 145
- IZUMRUDOV VLADIMIR A. ET AL.: 'Controllable stability of DNA-containing polyelectrolyte complexes in water-salt solutions' BIOPOLYMERS (NUCLEIC ACID SCIENCES) vol. 52, 1999, pages 94 - 108, XP002979408
- KABANOV ALEXANDER V. ET AL.: 'DNA interpolyelectrolyte complexes as a tool for efficient cell transformation' BIOPOLYMERS vol. 31, 1991, pages 1437 - 1443, XP000918675
- ZELIKIN ALEXANDER N. ET AL.: 'Polyelectrolyte complexes formed by calf thymus DNA and aliphatic ionenes: Unexpected change in stability upon variation of chain length of ionenes of different charge density' MACROMOL. BIOSCI. vol. 2, no. 2, 2002, pages 78 - 81, XP002979409
- RAMSDEN D.K. ET AL.: 'Flocculation of cellular material in complex fermentation medium with the flocculant poly(diallyldimethylammonium chloride)' BIOTECHNOLOGY TECHNIQUES vol. 12, no. 8, August 1998, pages 599 - 603, XP002979410
- DATABASE BIOSIS [Online] CIHLAR TOMAS ET AL.: 'Efficient separation of natural ribonucleotides by low-.pressure anion-exchange chromatography', XP002984043 Database accession no. (PREV199396107533) & JOURNAL OF CHROMATOGRAPHY vol. 644, no. 2, 1993, pages 299 - 305

## Description

### Technical field

The present invention relates to a method of isolating nucleic acids, such as DNA and/or RNA, from a biological solution. More specifically, the present method utilises a precipitation agent whereby a complex is formed, either from a desired nucleic acid and said precipitation agent or from an undesired nucleic acid and said agent.

### Background

In the 1860s F. Miescher isolated an acidic structure from the cell nuclei that he termed nuclein and later nucleic acid. The biological function of this material was not discovered until nearly a century later when it was established that nucleic acid material, and specifically DNA, was responsible for carrying hereditary information. When the solution to the molecular structure of DNA was reported in 1953, a new era in biochemistry and biology began.

As is well known by now, nucleic acid are polymers with a high density of negatively charged phosphate groups in the chains. There are two classes of nucleic acids found in every living organism, namely ribonucleic acid (RNA) and deoxyribonucleic acid (DNA). Viruses, on the other hand, contain only one type, either RNA or DNA. The biological functions of nucleic acids include the storage, replication, recombination and transmission of genetic information. The DNA can be grouped into nuclear DNA, cytoplasmic DNA, plasmid DNA, mitochondrial DNA, chloroplast DNA and viral DNA. The RNA can on the other hand be grouped into messenger RNA, ribosomal RNA, transfer RNA, small nuclear RNA, viral RNA and subviral RNA.

The various kinds of nucleic acids are these days used primarily in scientific research, but to an increasing extent also within the pharmaceutical and diagnostic fields. Thus, nucleic acids are e.g. useful in biotechnological processes wherein protein products are expressed from nucleic acids in cells, such as recombinant cells, as tools in methods for genetic manipulation and more recently also for medical and diagnostic applications. In order to obtain a useful nucleic acid product, a careful purification scheme is required in order to eliminate undesired components, such as cell debris, contaminants, such as toxic substances, e.g. endotoxins, etc.

The most common way to isolate nucleic acids has hitherto been to use chromatography. Thus, presently available purification methods are based on ultrafiltration, high-pressure liquid chromatography (HPLC), or extraction of nucleic acid fragments from agarose gels in the presence of chaotropic salts by precipitation onto glass or silica gel particles.

However, for the separation of nucleic acid mixtures comprising for example a doublestranded DNA fragment and a smaller single-stranded oligonucleotide, these methods are useful only with low efficiency.

Other more recently suggested purification schemes are based on the charge of the nucleic acids. Since nucleic acids are polymers with a high density of negatively charged phosphate groups in the chains, they can be considered as polyanions. Thus, various methods wherein nucleic acids are precipitated have been disclosed.

A specific form of selective precipitation of DNA has been disclosed in US patent application 20020010145 (Willson et al.). More specifically, purification of DNA, preferably plasmid DNA, from a preparation obtained from cell lysate after precipitation with organic solvent followed by resolubilisation in low-ionic-strength buffer, by selective precipitation by addition of compactation agents, is suggested. RNA, which is commonly the major contaminant in DNA preparations, can be left in solution while the desired plasmid DNA is directly precipitated. The compactation agents are small molecules, such as spermine and spermidine. Since the disclosed precipitation only occurs at low salt concentrations, the suggested method is not applicable directly on a cell lysate. Thus, there is still a need within this field of simplified methods, whereby the total number of steps required to obtain a purified DNA product is lowered and hence the total cost reduced.

An alternative approach based on precipitation of nucleic acids is disclosed in USP 5,622,822 (Ekeze et al.) as methods for capture and selective release of nucleic acids using polyethylene imine and an anionic phosphate ester surfactant. More specifically, nucleic acids are made available e.g. for amplification after lysis by contacting the lysate with polyethylene imine to form a precipitate. The nucleic acids are then released from the precipitate with a strong base, and the released nucleic acids are kept in solution with an anionic phosphate ester surfactant. However, the treatment with a strong base is harsh on the structure of the nucleic acids, and accordingly the method cannot be used to precipitate e.g. plasmid DNA. Furthermore, since ethylene imine is not charged at all pH values, it is essential to perform the method at a controlled pH.

Thus, there is still a need of alternative methods, which are useful with all forms of nucleic acids and which are simpler to operate.

An alternative method of precipitation of nucleic acids is disclosed in EP 1,031,626 (Erbacher et al), wherein a method of stabilising and/or isolating nucleic acids in a biological sample using ammonium or phosphonium salts comprised of 1-24 repeating units is suggested. However, the precipitation is non-selective i.e. the method does not allow specific precipitation of a particular type of nucleic acid e.g. genomic DNA, plasmid DNA, RNA with the rest of nucleic acids remaining in solution.

Furthermore, in the last decade complexes of nucleic acids with polycations has drawn a huge and increasing attention of scientists as vehicles for gene delivery. Since the vehicle should interact and bind strongly with negatively charged membranes of cells, it must be positively charged and soluble. These requirements are met only at a certain ratio of polycation/nucleic acid. Thus, contrary to biotechnological purification processes, wherein factors of simplicity makes the need to add a not too specific amount of complex binder attractive, it is in gene delivery methods a necessity to add a specific amount of polycation.

### Summary of the present invention

One object of the present invention is to provide a selective method of isolation of a nucleic acid from a solution that may contain other nucleic acids, proteins, other high molecular weight compounds, salts and other low-molecular weight substances, while leaving said other species in the solution. This is achieved by a method as defined in the appended claims.

Another object of the invention is to provide a method of isolation of a nucleic acid from a biological solution, which method is simple and suitable for large-scale operation. A related object of the invention is to provide such a method, which is also cost-effective especially in large-scale operation.

A further object of the invention is to provide a method of selective isolation of a nucleic acid, which is useful irrespective the pH of the solution and hence avoids use of pH-regulating agents such as acids and bases.

Yet another object is to provide a selective method of isolation of a nucleic acid as described above, which method allows precipitation thereof within a wide window of pH and salt concentrations and which is not sensitive to addition of an excess of precipitating agent.

A specific object of the invention is to provide a selective method of isolation of the nucleic acid plasmid DNA without having any essential affect on the plasmid's supercoiled structure. A particular objective is to provide a selective isolation method for plasmids available in a clarified alkaline lysate containing high concentrations of salts and RNA.

Further embodiments and advantages of the invention will appear from the following detailed description of the invention and from the experimental part.

### Brief description of the drawings

Figure 1 is a solubility diagram that illustrates the solubility of the complexes formed according to the invention by polycationic precipitating agents with different plasmids or RNA versus different charge ratios, [+] / [-].
Figure 2 shows a chromatographic analysis of the result of a model experiment with plasmid DNA and RNA performed in 1 M potassium acetate, pH 5.5.

Figure 3 shows the results from agarose gel electrophoresis obtained by the subsequent additions of polycationic precipitating agent to the same plasmid DNA containing clarified alkaline lysate.
Figure 4 shows the results from agarose gel electrophoresis illustrating how plasmid DNA can be selectively precipitated from a two-fold diluted clarified alkaline lysate.
Figure 5 is a plot of the percentage of nucleic acid in solution (Y-axis) versus the salt concentration (X-axis), which shows the dependence of DNA portion remaining in the supernatant of DNA /PDMDAAC (1), DNA/2,5-ionene bromide (2) and DNA/10,10-ionene bromide (3) systems on the concentration of the external salt.
Figure 6 shows the soluble nucleic acid versus charge ratio at different salt concentrations.

### Definitions

The term "biological solution" is used herein for any solution wherein biological material such as nucleic acids can be present. Thus, the term includes aqueous solutions, such as buffers, cell lysates, etc.
The term "nucleic acid" as used herein includes any form of nucleic acid, such as discussed in the section Background of the present specification.
The term "polycation" is used herein interchangeable with the term "polycationic precipitating agent".
A "polycation of integral type" denotes a molecule wherein the quaternary amine group is a part of the polymer chain, while the term "polycation of pendant type" denotes a molecule wherein the quaternary amine group is pendant from said chain.
The "charge ratio" is defined as [+] / [-]where [+] is the concentration of quaternary amino groups in the polycation and [-] is the concentration of phosphate groups in plasmid DNA.
The term "insoluble" which is used herein to denote a precipitation or a complex means a precipitation or complex, which can be separated from the solution wherein it has been formed by ordinary centrifugation.

### Detailed description of the invention

A first aspect of the present invention is a method of isolating a nucleic acid from a biological solution, which method comprises to selectively precipitate the desired nucleic acid by adding a polycationic precipitating agent to the solution and allowing it to form an insoluble complex with said nucleic acid, wherein the precipitating agent is a highly charged linear polymer that comprises quaternary amino groups. The method is selective in the respect that the desired nucleic acid is precipitated, while other nucleic acids as well as other molecules in the solution are not precipitated. Preferably, said agent is added in the presence of salt in such an amount that the charge ratio [+] / [-] between the polycationic precipitating agent and the nucleic acid is ≥ about 0.5 during the precipitation. As will be discussed in more detail below, the salt concentration is preferably controlled during the precipitation. In an advantageous embodiment, said charge ratio is ≥ about 0.9, preferably ≥ 1, during the precipitation.

Thus, the precipitate formed according to the invention is also known as an insoluble polyelectrolyte complex and the precipitating agents used herein are synthetic or natural polycations. Soluble polyelectrolyte complexes have previously mainly been utilised in studies e.g. of gene delivery, wherein their properties have been interpreted and predicted, rather than for purification in industrial scale bioprocesses. The polyelectrolyte complex, which is insoluble under the conditions used for precipitation, can be redissolved or even destroyed completely to form individual components. This is accomplished by adapting the conditions of salt concentration and/or pH in the solution, as will be discussed in more detail below.

In the present method, the desired nucleic acid is a plasmid.

The biological solution from which the desired nucleic acid is isolated can be any solution, which does not have any harmful impact on the nucleic acid and wherein the charge of the nucleic acid is essentially intact. Thus, in one embodiment, the biological solution is a cell lysate. The lysate can be a result of mechanical cell disruption. Alternatively, the lysate can be a clarified alkaline lysate, prepared by treating cells with a detergent-containing, strongly alkaline reagent followed by neutralization and centrifugation, yielding a nucleic acid-containing solution with a high salt concentration. Thus, one major advantage of the present method is that it is applicable also on solutions wherein the salt concentration is high.

Illustrative examples of the ability of the method to handle alkaline lysates are given in Example 9, 10 and 12. In these examples the salt concentration is very high during the nucleic acid precipitation step, namely around 0.6M with respect to potassium and 1M with respect to acetate. This salt concentration is much higher than those described in the references given in the background section.

In Example 9 and 10 the clarified lysate prepared according to Example 2 was first subjected to a pre-treatment (Example 2.1), to improve the polyelectrolyte precipitation step. The pre-treatment simply meant storing the clarified lysate at 4 °C for several days and then removing the spontaneously formed precipitate by centrifugation.

In Example 12 the clarified lysate was first subjected to an alternative pre-treatment (Example 2.2) to improve the polyelectrolyte precipitation step. The pre-treatment involved addition of hydrophobic zeolite. This alternative pre-treatment had the advantage of being very rapid in contrast to the above treatment that required storage for several days.

Hydrophobic zeolites used in Example 2.2 are known to adsorb SDS (sodium dodecyl sulphate), a compound added in the alkaline lysis step (Experiment 2). SDS or SDS in combination with other substances could be assumed to adversely interfere with the polyelectrolyte precipitation step and their removal should thus be beneficial. The choice of a zeolite with a suitable composition was governed by the instructions given by Eriksson and Green (The use of zeolite Y in the purification of intracellular accumulated proteins from genetically engineered cells. Biotechnol. Tech. 6 (1992) 239-244).

In an alternative embodiment, a pre-treatment step, such as a chromatographic separation, is applied on the biological solution before the precipitation according to the invention.

As mentioned above, the precipitating agent is a cationic, highly charged linear polymer that comprises quaternary amino groups, either as part of the polymer chain, known as integral polycations, or attached as substituents to the chain, known as pendant polycations. In this context, the term "highly charged" means that the ratio of polymer molecular weight (gram per mole)/polymer charge is less than 1000 and preferably less than 400. In a specific embodiment, said ratio is less than about 250, such as less than about 215. In one embodiment, the polycationic precipitating agent comprises at least about 25 positive charges i.e. quaternary amine groups. In a specific embodiment, the polycationic precipitating agent comprises at least about 50, more preferably at least about 500 and most preferably at least about 1000 positive charges i.e. quaternary amine groups. Usually, each repeating unit of the polymer will comprise one such amine group, and hence the numbers given above also applies to the number of repeating units in the precipitating agent used. Thus, in one embodiment, the precipitating agent is comprised of at least 1000 repeating units.

In one embodiment, the precipitating agent is a polymer that comprises about 1400 DP and exhibits a polymer molecular weight/polymer charge ratio of 160, namely poly(N,N'-dimethyldiallylammonium chloride) (DMDAAC), which is a commercially available product (Polysciences, Inc. Warrington, PA). In this embodiment, the precipitate, i.e. the insoluble polyelectrolytic complex, is formed in the region 0.5 ≤ [+]/[-] ≤ 10 and preferably in the region 0.7 ≤ [+]/[-] ≤ 5 depending on the salt concentration.

In another embodiment, the precipitating agent is a polymer that comprises an aliphatic ionene bromide that comprises about 80 DP and exhibits a polymer molecular weight/polymer charge ratio of 172.

In yet another embodiment, the precipitating agent is a polymer that comprises poly(N-alkyl -4-vinylpyridinium halides). Thus, in a specific embodiment, the precipitating agent is selected from the group that consists of poly(N-methyl -4-vinylpyridinium chloride), poly(N-ethyl-4-vinylpyridinium bromide) and poly(N-propyl-4-vinylpyridinium bromide). Some of them are commercially available (Polysciences, Inc. Warrington, PA). Accordingly, the preferred chain length is above DP 25 and the most preferred is above DP 100, the polymer molecular weight/polymer charge ratio is 214.

Thus, the precipitating agent is selected from the group that consists of poly(N,N'-dimethyldiallylammonium chloride, an aliphatic ionene bromide and a poly(N-alkyl-4-vinylpyridiniuxn halide).

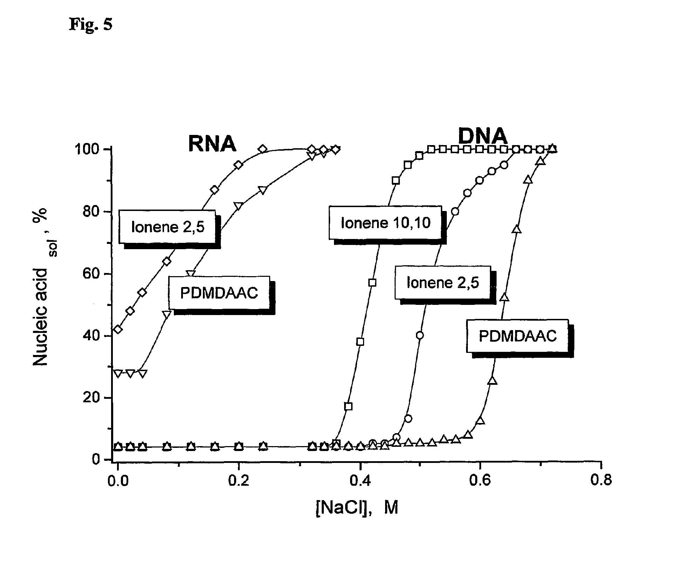

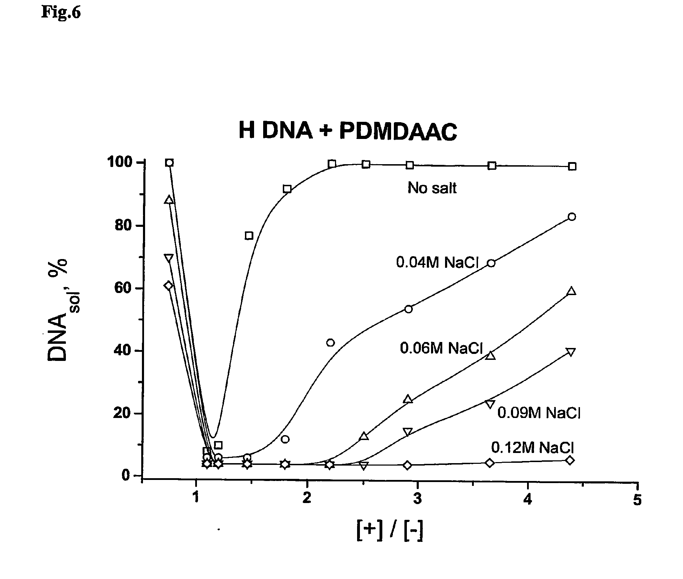

As mentioned above, in one embodiment, the salt concentration of the solution is controlled during the addition of the precipitating agent to allow the quantitative selective precipitation of the nucleic acid/polycation complex. In this context, the skilled person will have recognised that the optimal charge ratio for forming a specific complex will be shifted depending on the salt concentration in the biological solution during the precipitation. Thus, the unexpected finding that provides the basis for the present invention is that the present precipitation of nucleic acids can be obtained within a broad window of salt concentrations as compared to the prior art. This advantage appears clearly from Figure 5 of the present application. As mentioned above, previous selective precipitations of nucleic acids have been obtained at certain ratios. Contrary, the precipitation according to the invention can be performed in the presence of salt by adding an amount of polycationic precipitating agent to provide a number of positive charges which is either equivalent to or above the number of negative charges present on the nucleic acids. In this context, see figure 6 of the present application, wherein the advantages obtained at various charge ratios is illustrated for different salt concentrations. Accordingly, an advantage with the invention is that it will not entail any drawbacks to add an excess of polycationic precipitating agent, since it results in a higher charge ratio, which still allows effective precipitation. For reasons of simplicity, to ensure that a sufficient amount of polycationic precipitating agent has been added, excess is often added.

In the present invention, it is advantageous to determine the number of negative charges present on the nucleic acid in the biological solution before addition of precipitating agent. Accordingly, in one embodiment, the present method comprises a step of estimating the number of negative charges in the sample before addition of the precipitating agent. The skilled person in this field can easily determine the number of negative charges in a sample comprising nucleic acids according to standard methods, see e.g. example 6 below. In brief, such an estimation will e.g. include the steps of measuring the absorbance at 260 nm of a sample of a solution that contains DNA in order to determine the number of phosphate groups according to the well known formula A=ε*c*l, wherein A is absorbance, ε is the extinction coefficient and 1 is the length that the light travels across the cuvette. The extinction coefficient for DNA is 6500 M⁻¹cm⁻¹ (Olins, D. E.; Olins, A. L.; Von Hippel, P. H. Journal of Molecular Biology 1967, 24, 157-176). The concentration of negative charges of the DNA sample is determined by dividing the absorbance value with the extinction coefficient. The resulting value is the total concentration of negative charges. A sufficient estimation can be made for RNA using the same extinction coefficient. If the solution that it is desired to analyse contains unknown amounts of nucleic acid(s), as e.g. a lysate, a small sample is conveniently taken and run on an analytic column in order to separate DNA from RNA.

In one embodiment, the present method also includes to recover the desired nucleic acid by dissolving the formed precipitate by further addition of a salt. Thus, in one embodiment, the present method also comprises to recover the desired nucleic acid from the precipitate so formed by separating the precipitate from the solution and subsequent dissolution of the precipitate whereby a soluble complex is formed. In such a soluble complex, the precipitating agent is bound to the nucleic acid, but not sufficiently firmly to allow isolation thereof by ordinary centrifugation. Thus, in a specific embodiment, the present method also comprises to destruct the polyelectrolyte complex by addition of a salt whereby the desired nucleic acid is present as free in solution. In such a destructed complex, essentially no interaction occurs between the polymers of opposite charges, and both the polycationic precipitating agent and the nucleic acids exist separately/free in solution. Such free nucleic acids are conveniently recovered by chromatography, electrophoresis or any other well-known method. Even though the skilled person in this field will appreciate that the exact values of salt concentration will depend on the other parameters such as the nature and amount of the precipitating agent, this kind of complexes are destructed at increased salt concentrations. Accordingly, in a specific embodiment, the dissolution and destruction of the precipitation is performed at a salt concentration above 0.5 M, preferably above 3 M, depending on the charge ratio [+] / [-] and salt nature.

The salt can be virtually any salt that is well known in the field of chromatography and commonly used for desorption of ion exchangers, such as sodium chloride, potassium chloride, ammonium acetate, potassium acetate etc. The only requirements of the salts used in the present method are that they are capable of displacing the polycationic precipitation agent from the complex, thereby freeing the nucleic acids, and that they have no harmful impact on the desired nucleic acids.
Optionally, the precipitate can be washed, e.g. with water or a suitable buffer, before dissolution thereof.

Another aspect of the invention is the use of a method according to the invention for isolating nucleic acids that have been subjected to modification reactions. Thus, such nucleic acids can for example be nucleic acid fragments.

A last aspect of the invention is a kit that comprises sufficient materials for performing the method according o the invention, e.g. precipitating and optionally salt to be added in separate compartments together with written instructions as regards how to perform such a method.

### Detailed description of the drawings

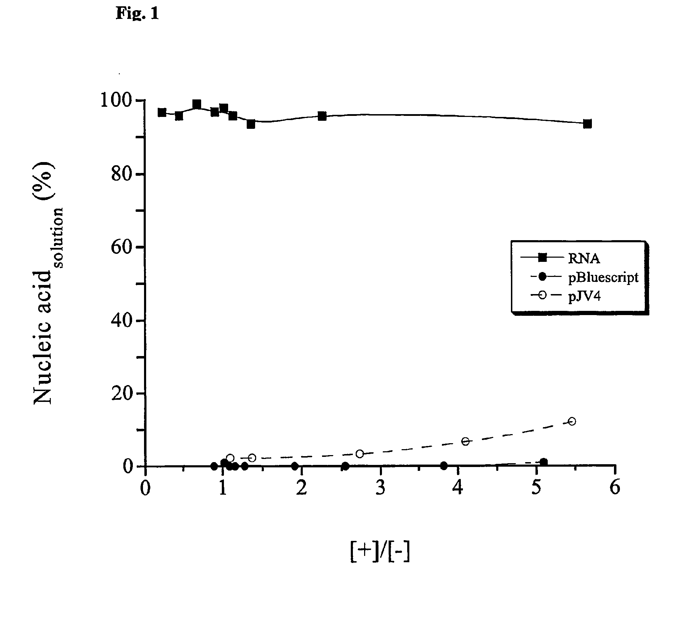

Figure 1 is a solubility chromatogram that illustrates how the solubility of the complexes formed by polycationic precipitating agent with different plasmids or RNA at different charge ratios, i.e. different [+] / [-], were studied at 1 M potassium acetate (see example 7). The data obtained on studying mixtures of either plasmid DNA or RNA with polycationic precipitating agent are shown as the portion of DNA remaining in supernatant versus [+] / [-].

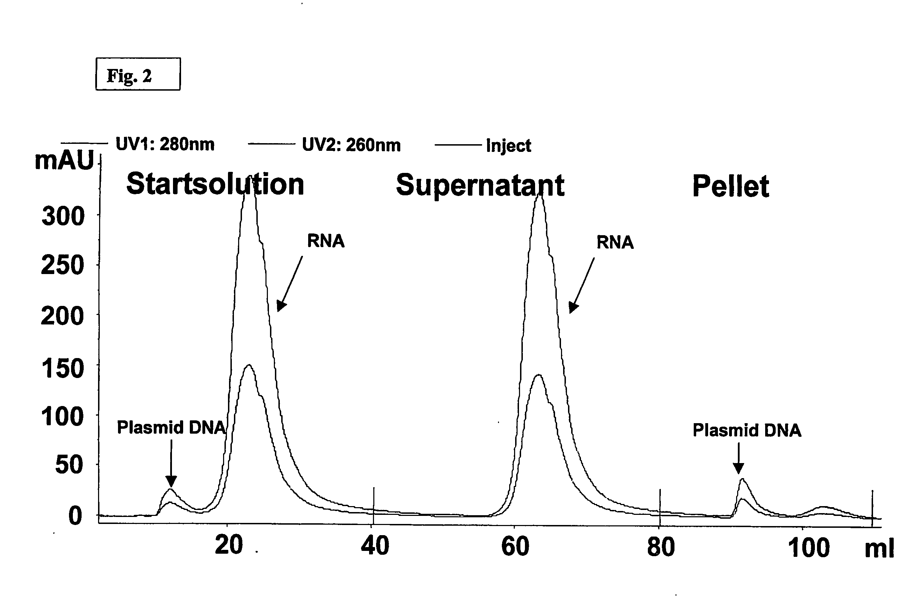

Figure 2 shows a chromatographic analysis of the results of a model experiment with plasmid DNA and RNA was performed at 1 M potassium acetate, according to Example 8 below. Similar conditions as in a clarified alkaline lysate were used, i.e. amount of RNA >> amount of plasmid DNA. The ratio between polycationic precipitating agent and plasmid DNA was 1.4, i.e. [+]/[-] = 1.4.

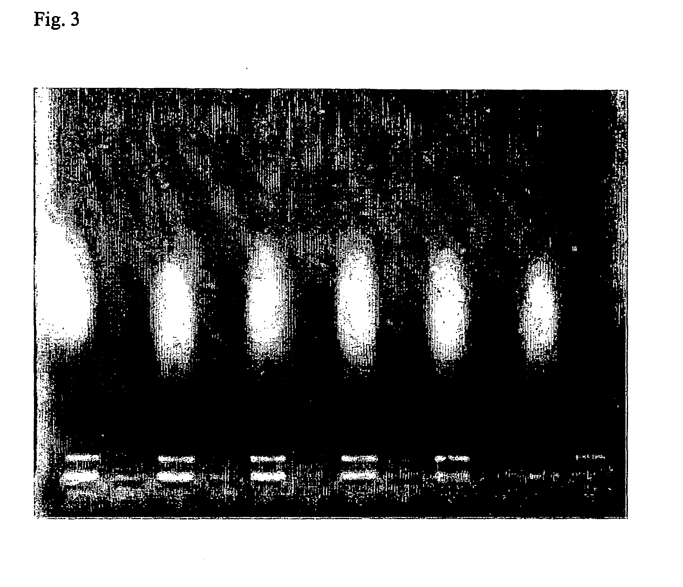

Figure 3 shows the results from an agarose gel electrophoresis obtained by the subsequent additions of polycationic precipitating agent to the same plasmid containing solution in accordance with Example 9 below. To a two-fold diluted plasmid DNA containing clarified alkaline lysate polycationic precipitating agent solution was added, corresponding to [+] / [-] = 1. After precipitation the supernatant was transferred to a new test tube and polycationic precipitating agent of the same amount was again added. This procedure was repeated four times. The blank is a two-fold diluted lysate, which was also centrifuged and resulted in a small pellet that did not contain any plasmid DNA. [+] / [-] = 5 means that the amount of polycationic precipitating agent [+] / [-] = 1, has been added subsequently five times to same solution. S denotes supernatant and P for pellet. The pellets were dissolved in 2 M potassium acetate. The samples were analysed on agarose gel electrophoresis as described in Example 5 below. From Figure 3, it is evident that the method according to the invention at [+] / [-] = 5 resulted in selective precipitation of plasmid DNA. As is easily realised by the skilled person, by adding polycationic precipitating agent of course some dilution in each step was achieved, which is seen in Figure 3 as a decrease in the intensity of the bands on the agarose gel.

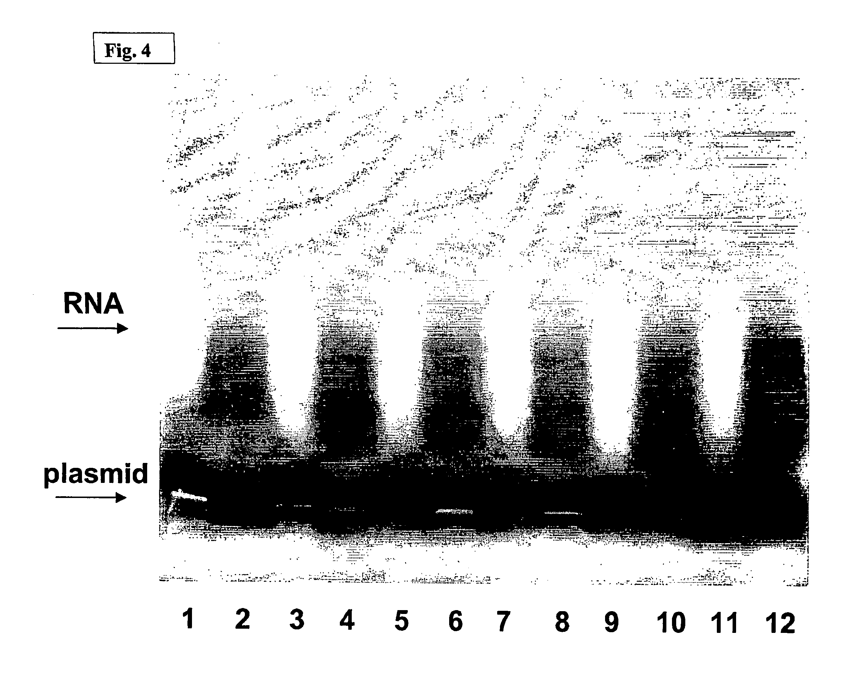

Figure 4 illustrates the result of agarose gel electrophoresis of a clarified alkaline lysate which was two-fold diluted and polycationic precipitating agent was added, corresponding to [+]/[-] = 4. After precipitation no RNA or plasmid DNA is detected in the dissolved pellet (Lane 2). Five identical samples were prepared according to example 10 in test tubes. After precipitation the five supernatants were transferred to new tubes. To the five supernatants was added different volumes of 2 mM (based on the corresponding monomer concentration) polycation solution according to the invention, corresponding to [+] / [-] = 1, 2, 3, 4, 5 respectively. As a blank the solution of clarified alkaline lysate and distilled water was used. All pellets were redissolved in 1 ml 2 M potassium acetate, pH 5.5. The samples were analysed on agarose gel electrophoresis as described in Example 5. The results from the second precipitation with [+]/[-] = 4 + 1 is given in Lane 3 - 4, where 3 is the supernatant and 4 is the dissolved pellet. The results from the second precipitation with [+]/[-] = 4 + 2 is given in Lane 5-6 and, 4+3 in lanes 7-8, 4+4 in lanes 9-10 and 4+5 in lanes 11-12. Lane 1 shows the two-fold diluted clarified alkaline lysate. The two-step precipitation resulted in a partial precipitation of the plasmid DNA at [+] /[-] = 4 + 1, but a complete and selective precipitation in all the rest.

Figure 5 shows the dependence of DNA portion remaining in the supernatant of DNA /PDMDAAC (1), DNA/2,5-ionene bromide (2) and DNA/10,10-ionene bromide (3) systems on the concentration of the external salt. Other conditions are the same as in Fig.6.
Figure 6 shows the dependence of DNA portion remained in the supernatant of DNA/PDMDAAC system on the composition ϕ = [+] / [-] in the absence of external salt (1) and in the presence of different NaCl concentration of M: 0.04 M (2), 0.06 M (3), 0.09 M (4) and 0.12 M (5). 0.02 M Tris-HCl, pH 9.0,25 °C.

### EXPERIMENTAL PART

The following examples are provided for illustrative purposes only and are not to be construed as limiting the present invention as defined by the appended claims. All references given below or elsewhere in the present application are hereby included by reference.

### Materials

Fermentation media:
30 g Tryptone soya broth / L (OXOID)
10 g Yeast extract / L (Gistex)
10 g Dextrose / L
100 mg Ampicillin / L (Sigma, St. Louis, MO, USA)

### Expression plasmid and bacterial strain:

*E. coli* XL1 Blue harbouring the plasmid pBluescript II KS (+/-) 2.9 kbp having an insert of a xylanase gene from Rhodothermus marinus (3 kbp) giving a total plasmid size of 5.9 kbp (Eva Nordberg Karlsson, Xylan degradation by the thermophilic bacterium Rhodothermus marinus: Characterization and function of a thermostable xylanase. Doctoral thesis, Department of Biotechnology, Lund University, Sweden, 1999, ISBN 91-628-3598-X).

### Example 1: Cell growth

E. Coli cells harbouring the plasmid was grown in a 500 ml shake flask containing 100 ml fermentation media (37°C, 160 rpm, 9 h) to an optical density of 2 (OD_{600 nm}). 10 ml each of this overnight culture was used to inoculate four 500 ml shake flasks each containing 100 ml fermentation media and the cells were grown further for 9 h (37°C, 160 rpm) to an optical density of 6.5 (OD_{600 nm}). All of this culture (400 ml) was used to inoculate a 15 L fermentor (Electrolux) containing 10 L of fermentation media. The cells were grown for 8.5 h (37°C, 600 rpm) to an optical density of 12.5 (OD_{600 nm}). During fermentation the pH of the medium was kept at 7 by addition of 1 M NaOH and foam was inhibited by occasional addition of adekanol (Asahi Denka Kogyo K.K., Japan). The 10 L cell culture was pumped through sterile tubing into a 784 L fermentor (Belach bioteknik AB, Stockholm, Sweden) containing 400 L of fermentation media. The cells were grown for 10 h (37°C) to an optical density of 13 (OD_{600 nm}). During fermentation the pH of the medium was kept at 7 by addition of 5 M NaOH and foam was inhibited by addition of adekanol (control unit). 3.5 kg of dextrose in 8 L of water was also pumped into the fermentor during the cultivation (fed-batch, control unit). The agitation speed was controlled by the oxygen demand. The cells were harvested by centrifugation at 15 000 rpm in a Sharpless centrifuge operated with a feed rate of 1.2 L / min. The obtained cell paste was stored as 5, 25 and 300 g aliquots at - 80 °C.

### Example 2: Cell lysis

Cell lysis was performed by the alkaline lysis method as follows: 5 g of cell paste from Example 1 was defrosted and completely resuspended by gentle vortexing in 36 ml of 10 mM Tris-HCl, pH 8, 61 mM glucose, 50 mM EDTA. The cell suspension was transferred to a plastic beaker equipped with magnetic stirring and 78 ml of 0.2 M NaOH containing 1% SDS was added and the gentle stirring was continued for 7 minutes at room temperature. After this incubation period, 59 ml of cold (5°C) 3 M KAc, pH 5,5 was added and the solution was gently mixed by magnetic stirring for 20 minutes on an ice bath. The white precipitate formed was removed by 30 minutes centrifugation at 4°C at 10.000 rpm in a Sorvall GSA rotor. The supernatant was finally filtered through a nylon net (Falcon Cell Strainer, 35 µm pore size).

### Example 2.1 Pre-treatment of clarified lysate by storage at 4 °C

The clarified lysate from Example 2 was stored at 4 °C for 6 days. The formed precipitate was removed by 30 minutes centrifuagation at 4 °C at 10 000 rpm in a Sorvall GSA rotor.

### Example 2.2 Pre-treatment of clarified lysate by zeolite

Zeolite suspension (160 mg/ml) was prepared by mixing solid Zeolite Y (Zeolite Y, with a SiO₂ / Al₂O₃ mole ratio of 430, was obtained from Tosoh Co., Japan) with 25 mM Tris-HCl, pH 8 and NaCl. The final concentration of Tris-HCl was 2 mM and NaCl concentration was 0.2 M. The suspension was incubated at room temperature during gentle mixing for 20 min. 52.5 ml of the zeolite suspension was then added to 105 ml clarified lysate, prepared as described in example 2, mixed for 60 s and then centrifuged for 10 min at 13000 x g at 4°C. The supernatant was collected and stored in a refrigerator until used.

### Example 3: Preparation of plasmid DNA

Cell paste from Example 1 was defrosted and the plasmid DNA was purified by the Qiagen Plasmid Mega kit (Qiagen GmbH, Hilden, Germany) according to the manufacturer instruction.

A pure plasmid preparation (pJV4, 50 µg/ml) consisting of pUC 19 (2.7 kbp) having a 3.4 kbp insert (JV4-dmgA-demA gene) from *Streptococcus dysgalactiae* giving a total plasmid size of 6.1 kbp was obtained from Amersham Biosciences AB, Uppsala, Sweden.

### Example 4: Analysis by group separation on Sephacryl S-500 column

Sephacryl^{™} S-500 beads (Amersham Biosciences AB, Uppsala, Sweden) was packed into a XK 16/20 column (Amersham Biosciences AB, Uppsala, Sweden) and integrated to an ÄKTA^{™} explorer 10 system (Amersham Biosciences AB, Uppsala, Sweden). The column was equilibrated with 2 M KAc pH 5.5. 1 ml of the sample was injected and run at a flow rate of 1 ml/min (30 cm/h). The eluted peaks were detected at 260 and 280 nm.

### Example 5: Analysis by gel electrophoresis

Gel electrophoresis was performed on 0.7 % agarose gels in TBE buffer (0.089 M Tris-borate, pH 8.0, 2 mM EDTA). 15 µl of samples was loaded in each well and the samples were run at 60 V for 60 minutes on a Hoefer^{™} HE 33 Mini horizontal submarine unit (Amersham Biosciences AB, Uppsala, Sweden) powered by a electrophoresis power supply (EPS 301, Amersham Biosciences AB, Uppsala, Sweden). After the run the agarose gel was stained with ethidium bromide by soaking the gel in 100 ml TBE buffer containing 1.5 µg ethidium bromide/ml. The agarose gel was analysed and photographed using the gel documentation software Alphalmager 2200 v5.5 from Alpha Innotech Corporation (San Leandro, CA, USA).

### Example 6: Determination of the amount of negative charges on nucleic acids

The concentration of nucleic acid was determined by measuring the absorbance at 260 nm and assuming the molar extinction coefficient 6500 M⁻¹cm⁻¹ as calculated per one phosphate group. The concentration of nucleic acid is presented as concentration of phosphate groups, i.e. concentration of negative charges.

### Example 7: Solubility of complexes formed by polycationic precipitating agent, poly(N,N'-dimethyldiallylammonium) chloride, with plasmid DNA or RNA

0.1 ml plasmid DNA solution (pJV4 or pBluescript, 6 kbp), prepared as described in Example 3, or 0.1 ml RNA solution (from bakers yeast, Sigma, St. Louis, MO, USA) at a concentration of 0.05 mg/ml was mixed with a 3 M potassium acetate solution, pH 5.5 and polycation solution (2-50 µl at a concentration of 2 mM (based on the corresponding monomer concentration). The final volume was 1.0 ml and the final potassium acetate concentration 1 M. After vigorous shaking for at least one minute and centrifugation at 14100 g for 10 min, the formation of polyelectrolyte complexes was monitored by measuring the amount (absorbance at 260 nm) of residual nucleic acid in the supernatants. The results are presented in Figure 1 as nucleic acid remaining in solution versus the ratio of charges, [+] / [-].

### Example 8: Separation of plasmid DNA from an "artificial" clarified alkaline lysate by poly(N,N'-dimethyldiallylammonium) chloride

0.75 ml plasmid DNA solution (pJV4, prepared as described in Example 3) at a concentration of 0.05 mg/ml, 0.075 ml RNA solution (from bakers yeast, Sigma, St. Louis, MO, USA) at 10 mg/ml, 0.5 ml 3 M potassium acetate solution, pH 5.5 and 0.175 ml distilled water were mixed vigorously with 0.08 ml 2 mM (based on the corresponding monomer concentration) polycationic precipitating agent (corresponds to [+] / [-] = 1.4). After centrifugation at 14100 g for 10 min the supernatant was removed and the pellet dissolved in 1.5 ml 2 M potassium acetate, pH 5.5. The analysis was performed on start solution (plasmid DNA, RNA and buffer without added polycationic precipitating agent), supernatant and dissolved pellet according to the method described in Example 4. The result is shown in Figure 2.

### Example 9: Determination of optimal charge ratio when separating plasmid DNA from a clarified alkaline lysate by poly(N,N'-dimethyldiallylammonium) chloride

To 0.5 ml of the lysate, prepared according to Example 2.1, was added 0.5 ml of distilled water. To this solution was added 4.7 µl 2 mM (based on the corresponding monomer concentration) polycationic precipitating agent (corresponding to [+] / [-] = 1). After vigorous shaking and centrifugation at 14100 g for 10 min the supernatant was transferred to a new tube and the same amount of polycationic precipitating agent was again added. This procedure was four times repeated. As a blank the solution of plasmid DNA and distilled water was used. All pellets were dissolved in 1 ml 2M potassium acetate, pH 5.5. The samples were analysed on agarose gel electrophoresis as described in Example 5.

### Example 10: Separation of plasmid DNA from a clarified alkaline lysate using poly(N,N'-dimethyldiallylammonium) chloride

To 0.5 ml of a clarified alkaline lysate, which had been prepared according to Example 2.1, 0.480 ml of distilled water was added. Five identical samples were prepared in test tubes by to each adding 18.9 µl 2 mM (based on the corresponding monomer concentration) polycation solution (corresponding to [+] / [-] = 4). After vigorous mixing and centrifugation at 14100 g for 10 min, the five supernatants were transferred to new tubes. To the supernatants 4.7, 9.7, 14.2, 18.9 and 23.6 µl (corresponding to [+] / [-] = 1, 2, 3, 4, 5) polycation solution respectively at a concentration of 2 mM was added. As a blank the solution of clarified alkaline lysate and distilled water was used. All pellets were dissolved in 1 ml 2 M potassium acetate, pH 5.5. The samples were analysed on agarose gel electrophoresis as described in Example 5.

### Example 11: Analysis by anion-exchange chromatography on MiniQ Column

The recovery of supercoiled plasmid was determined by analytical ion-exchange chromatography on a MiniQ column (4.6 x 50 mm) integrated to an ÄKTA^{™} explorer system (all obtained form Amersham Biosciences, Uppsala, Sweden) and equilibrated with 25 mM Tris-HCl, pH 8 containing 0.5 M NaCl. To avoid the interference of RNA in the quantitation of plasmid in clarified alkaline lysate samples, these were incubated with RNase (100 µg/ml) for 15 minutes prior to the analysis. Samples of 100 µl were injected on the column and adsorbed nucleic acids were then eluted by applying a gradient from 0.5 to 0.8 M NaCl in 18 column volumes. The eluate from the column was monitored by UV absorbance at 260 nm and 280 nm. The analysis was performed at a flow rate of 0.4 ml/min.

### Example 12. Selective precipitation of plasmid DNA from a zeolite treated clarified alkaline lysate

To the zeolite-treated lysate (Example 2.2), 11.1 ml 2 mM PDMDAAC (based on the corresponding monomer concentration) was added. The final volume was set to 210 ml by the addition of water. The sample was mixed for about 60 s and centrifuged for 10 min at 14100 g (15-20°C). After decanting the supernatant the pellet was re-dissolved in 5 ml 25 mM Tris-HCl, pH 8 including 2 M NaCl. Chromatography analysis (example 4 and 11) was performed to determine the content of plasmid DNA and RNA while the BCA method was used for proteins (Sigma procedure NO. TPRO-562). The clarified lysate, the zeolite-treated lysate and the re-dissolved pellet were analyzed by these methods. The results from this analysis are displayed in Table 1.

**Table I. Analysis of precipitation of plasmid DNA from a clarified lysate. (All values are given as per cent of initial amount).**

| | Plasmid DNA | **RNA** | **Protein** |
|---|---|---|---|
| Clarified lysate | 100 | 100 | 100 |
| Zeolite-treated lysate | 100 | 55 | 40 |
| Re-dissolved Pellet | 77 | 4 | < 10 |

Plasmid DNA and RNA was analysed by size exclusion chromatography (Example 4) or by anion exchange chromatography (Example 11). Protein was analysed by the BCA method (Sigma procedure NO.TPRO-562).

## Claims

1. A method of isolating a plasmid from a biological solution, which method comprises to selectively precipitate the desired nucleic acid by adding a polycationic precipitating agent to the solution and allowing it to form an insoluble complex with said nucleic acid, wherein the precipitating agent is selected from the group that consists of poly(N,N'-dimethyldiallylammonium chloride), an aliphatic ionene bromide and a poly(N-alkyl -4-vinylpyridinium halide).

2. A method according to claim 1, wherein the biological solution is a cell lysate.

3. A method according to claim 2, wherein the cell lysate is an alkaline cell lysate.

4. A method according to any one of the preceding claims, wherein the salt concentration of the solution is controlled during the addition of the precipitating agent to allow the quantitative selective precipitation of the nucleic acid/polycation complex.

5. A method according to any one of the preceding claims, which also comprises to recover the desired nucleic acid from the precipitate so formed by separating the precipitate from the solution and subsequent dissolution and/or destruction of the complex.

6. A method according to claim 5, wherein the polyelectrolyte complex is dissolved and/or destructed by addition of a salt to free the desired nucleic acid in the solution.

7. A method according to claim 6, wherein the dissolution and/or destruction of the complex is performed at a salt concentration above 0.5 M depending on the charge ratio [+] / [-] and salt nature.

8. A method according to any one of the preceding claims, wherein the plasmids, have been subjected to modification reactions.

## Patentansprüche

1. Verfahren zum Isolieren eines Plasmids aus einer biologischen Lösung, wobei das Verfahren umfasst, selektives Ausfällen der erwünschten Nukleinsäure durch Zugeben eines polykationischen Ausfällungsmittels zur Lösung und ihr Ermöglichen, einen unlöslichen Komplex mit der Nukleinsäure zu bilden, wobei das Ausfällungsmittel ausgewählt ist aus der Gruppe, die besteht aus Poly(N,N'-dimethyldiallylammoniumchlorid), ein aliphatisches Ionen-bromid und ein Poly(N-alkyl-4-4-vinylpyridiniumhalogenid).

2. Verfahren nach Anspruch 1, wobei die biologische Lösung ein Zelllysat ist.

3. Verfahren nach Anspruch 2, wobei das Zelllysat ein alkalisches Zelllysat ist.

4. Verfahren nach einem der vorstehenden Ansprüche, wobei die Salzkonzentration der Lösung geregelt wird während der Zugabe des Ausfällungsmittels, um die quantitative selektive Ausfällung des Nukleinsäure/Polykationkomplexes zu ermöglichen.

5. Verfahren nach einem der vorstehenden Ansprüche, das auch umfasst, die erwünschte Nukleinsäure aus dem so gebildeten Präzipitat wiederzugewinnen durch Trennen des Präzipitats von der Lösung und nachfolgender Auflösung und/oder Zerstörung des Komplexes.

6. Verfahren nach Anspruch 5, wobei der Polyelektrolytkomplex aufgelöst und/oder zerstört wird durch Zugabe eines Salzes, um die erwünschte Nukleinsäure in der Lösung freizusetzen.

7. Verfahren nach Anspruch 6, wobei die Auflösung und/oder Zerstörung des Komplexes ausgeführt wird bei einer Salzkonzentration über 0,5 M, abhängig von dem Ladungsverhältnis [+]/[-] und der Salznatur.

8. Verfahren nach einem der vorstehenden Ansprüche, bei dem die Plasmide Modifikationsreaktionen unterzogen worden sind.

## Revendications

1. Procédé d'isolation d'un plasmide à partir d'une solution biologique, lequel procédé comprend la précipitation sélective de l'acide nucléique désiré en ajoutant un agent de précipitation polycationique à la solution et en l'amenant à former un complexe insoluble avec ledit acide nucléique, dans lequel l'agent de précipitation est sélectionné à partir du groupe qui se compose du poly(chlorure de N,N'-diméthyldiallylammonium), d'un bromure ionène aliphatique et d'un poly(halogénure de N-alkyl-4-vinylpyridinium).

2. Procédé selon la revendication 1, dans lequel la solution biologique est un lysat cellulaire.

3. Procédé selon la revendication 2, dans lequel le lysat cellulaire est un lysat cellulaire alcalin.

4. Procédé selon l'une quelconque des revendications précédentes, dans lequel la concentration en sel de la solution est contrôlée pendant l'ajout de l'agent de précipitation afin de permettre la précipitation sélective quantitative du complexe acide nucléique/polycation.

5. Procédé selon l'une quelconque des revendications précédentes, qui comprend aussi la récupération de l'acide nucléique désiré à partir du précipité ainsi formé en séparant le précipité de la solution, et la dissolution et/ou la destruction consécutive du complexe.

6. Procédé selon la revendication 5, dans lequel le complexe polyélectrolyte est dissous et/ou détruit par ajout d'un sel afin de libérer l'acide nucléique désiré dans la solution.

7. Procédé selon la revendication 6, dans lequel la dissolution et/ou la destruction du complexe est exécutée à une concentration en sel supérieure à 0,5 M en fonction du rapport de charge [+]/[-] et de la nature de sel.

8. Procédé selon l'une quelconque des revendications précédentes dans lequel les plasmides ont été soumis à des réactions de modification.
